# EUROPEAN PATENT SPECIFICATION

(11) **EP 3 957 318 B1**
(45) Date of publication and mention of the grant of the patent: **21.08.2024**
(21) Application number: 19940174.6
(22) Date of filing: 07.08.2019
(51) Int. Cl.: A61B 18/00, A61F 7/00, A61N 1/40, A61N 7/02, A61K 38/19, A61P 35/04, C07K 14/52, A61P 43/00, A61P 35/00

(54) **PHARMACEUTICAL COMPOSITION**
PHARMAZEUTISCHE ZUSAMMENSETZUNG
COMPOSITION PHARMACEUTIQUE

(43) Date of publication of application: 23.02.2022
(73) Proprietor: Hirata Corporation, Kumamoto-shi, Kumamoto 861-0198 (JP)
(72) Inventor: KANEGASAKI, Shiro, Tokyo 107-0052 (JP)
(74) Representative: Müller-Boré & Partner Patentanwälte PartG mbB
(86) International application number: PCT/JP2019/031138
(87) International publication number: WO 2021/024419

(56) References cited:
- JP-A- 2019 034 164
- KR-A- 20170 046 373
- PING CHEN ET AL: "Synergistic antitumor effect of CXCL10 with hyperthermia", JOURNAL OF CANCER RESEARCH AND CLINICAL ONCOLOGY, SPRINGER, BERLIN, DE, vol. 134, no. 6, 8 December 2007 (2007-12-08), pages 679 - 687, XP019630699, ISSN: 1432-1335
- N. IIDA ET AL: "Antitumor Effect after Radiofrequency Ablation of Murine Hepatoma Is Augmented by an Active Variant of CC Chemokine Ligand 3/Macrophage Inflammatory Protein-1", CANCER RESEARCH, vol. 70, no. 16, 15 August 2010 (2010-08-15), pages 6556 - 6565, XP055019917, ISSN: 0008-5472, DOI: 10.1158/0008-5472.CAN-10-0096
- RYAN SLOVAK ET AL: "Immuno-thermal ablations - boosting the anticancer immune response", JOURNAL FOR IMMUNOTHERAPY OF CANCER, BIOMED CENTRAL LTD, LONDON, UK, vol. 5, no. 1, 17 October 2017 (2017-10-17), pages 1 - 15, XP021249814, DOI: 10.1186/S40425-017-0284-8
- BIJGAART RENSKE J VAN DEN ET AL: "Thermal and mechanical high-intensity focused ultrasound: perspectives on tumor ablation, immune effects and combination strategies", CANCER IMMUNOLOGY IMMUNOTHERAPY, SPRINGER, BERLIN/HEIDELBERG, vol. 66, no. 2, 1 September 2016 (2016-09-01), pages 247 - 258, XP036145808, ISSN: 0340-7004, [retrieved on 20160901], DOI: 10.1007/S00262-016-1891-9
- YASUNORI AKUTSU, HIROSHI TAMURA, KENTARO MURAKAMI, SHINI, AKIKO SUGANAMI, HISAHIRO MATSUBARA: "WS1-5: Basic and clinical of immunotherapy with Abscopal Effect according to new thermal treatment", THERMAL MEDICINE, vol. 29, no. Suppl., 30 November 2012 (2012-11-30) - 31 August 2013 (2013-08-31), JP, pages 84, XP009531753, ISSN: 1882-2576

## Description

The present invention relates to a pharmaceutical composition comprising a chemokine for use in suppressing or preventing cancer metastasis, wherein the pharmaceutical composition is administered to a patient in combination with hyperthermia, wherein the cancer is locally heated in the hyperthermia.

Cancers including carcinomas, which occur in epithelia, and non-epithelial sarcomas are malignant neoplasms that are generated due to changes in a plurality of genes caused by DNA disorders and continue to abnormally proliferate. Cancers that have grown to a certain size also infiltrate surrounding tissues and organs. Then, cancers spread to tissues distant from primary lesions of the cancers via blood vessels and lymphatic vessels by distant metastasis, and gain a function of proliferating again at the metastatic lesions. When a person is clinically diagnosed with a cancer, such a cancer has already grown to a size larger than or equal to a certain size, and it is often thought that the cancer spreads to many organs in addition to the detected metastatic lesion by micrometastasis formation. Hematogenous metastasis of cancer cells occurs through a plurality of steps including separation of cancer cells from the primary lesion thereof, infiltration into surrounding tissues (interstitial tissues), entrance into blood vessels, adhesion to vascular endothelial cells in a target organ, exudation from the blood vessels, and engraftment to the target organ and reproliferation therein. Accordingly, a cancer at a metastatic lesion has gained new characteristics that are not in common with the characteristics of a cancer at a primary lesion, and it is observed that a large number of molecules related to such characteristics are expressed in the cancer at a metastatic lesion.

A living organism has a biological defense mechanism such as immunity for removing abnormal cells. However, cancers evade such a defense mechanism and thus become malignant, and as a result, metastatic cancers also have tolerance for the biological defense mechanism. The mechanism for allowing cancer cells to evade immunity is as follows, for example: immune checkpoint proteins such as PD-L1 are expressed and thus responses of defensive cells (T-lymphocytes that express corresponding PD-1) are suppressed and stopped. Furthermore, cancer cells promote the expression of CTLA-4, which suppresses the activation of T-cells, on T-lymphocytes, and thus an immunosuppressive function is exhibited.

A systemic treatment method that consists principally of chemotherapy is commonly used as a cancer treatment method. Conventionally, in typical chemotherapy, drugs that function to target a difference in the proliferation rate between cancer cells and normal tissue cells have been used as a first-choice drug. However, in a systemic treatment method, such drugs also cause damage to tissue cells that proliferate faster than cancer cells in a living organism. Side effects of recent cancer therapeutic drugs are reduced by improving the doses and administration methods thereof, but a biological defense mechanism that is mainly based on leukocytes is particularly problematic. In such a biological defense mechanism, hematopoietic stem cells in bone marrow produce about 700 to 1000 neutrophils (one type of leukocyte) per day, for example. Moreover, activated lymphocytes can also divide at least once every 8 hours, and continue to double every 8 hours. Accordingly, if such a drug is administered as a first-choice drug, leukocyte cells including these neutrophils and lymphocytes will be damaged first. Therefore, many chemotherapies have limitations in that a biological defense mechanism does not function and a backup thereof cannot be expected, and as a result, some surviving cancer cells will become drug-resistant cancer cells.

Nowadays, in addition to these chemotherapies, systemic therapies based on the development of immunology with which the activities of checkpoint proteins are suppressed have also begun to be widely attempted. It has been considered that such immunotherapies have relatively fewer side effects, but many side effects caused by suppression of the entire immune function have been actually reported. The number of lymphocytes that can be recruited from peripheral blood to combat a cancer containing 10⁹ cells per gram is limited, and therefore, monotherapies that depend only on individual defense mechanisms essentially exhibit insufficient effects.

On the other hand, surgical operations and irradiation are widely performed as local cancer treatment methods. However, the greatest shortcoming of local therapies is that cancers and the like that are present in sites other than a local treated site and surrounding infiltrated tissues due to distant metastasis are not treatment targets. The procedures of local therapies may be essentially incapable of being applied to certain sites due to various factors, or certain types of cancer cells or tissues that are less sensitive to a radiotherapy may be present. Accordingly, the procedures themselves are insufficient and thus the effects thereof are limited in many cases. Moreover, ordinary radiotherapies in which X-rays are used have a problem of common side effects caused by inflammation of normal tissues, and also have a problem in that the functions of a biological defense mechanism are impaired due to a decrease in lymphocytes after irradiation.

Hyperthermia that is less likely to cause serious side effects is known as another cancer treatment method. The following describes the mechanism of hyperthermia. The functions of blood vessels in a cancer site are impaired compared with those in a normal site. That is, in a cancer site, the heat dissipation function is deteriorated because blood vessels are not dilated even through heating and thus blood flow is not increased. As a result, when a region that includes a cancer site is heated, the temperature of the cancer site is particularly raised. Moreover, oxygen supply to a cancer site is limited, and therefore, lactic acid accumulates in the cancer site, which leads to deterioration in thermotolerance of cells. For these reasons, cancer cells are more sensitive to heating than normal cells.

In order to clinically use hyperthermia to treat cancers, there is demand to improve the effects of hyperthermia. For example, Patent Literature 1 discloses that the effects of hyperthermia are enhanced by administering 5-aminolevulinic acids.

On the other hand, the inventor of the present invention reported that the effects of cancer radiation treatment could be improved by performing the cancer radiation treatment while using a chemokine CCL3 derivative (Non-Patent Literature 1).

In this context, KR 2017 0046373 A describes a composition for preventing or treating cancer comprising alarmin and eMIP polypeptide as an active ingredient. Further, Chen et al. (Chen, P. et al.; J Cancer Res Clin Oncol, 134; 2008; pp. 679-687) describes a synergistic antitumor effect of CXCL10 with hyperthermia. Furthermore, Iida et al. (Iida, N. et al.; Cancer Res, 70(16); 2010; pp. 6556-6565) describes the augmentation of the antitumor effect after radiofrequency ablation of murine hepatoma by an active variant of CCL3/MIP1α.

### PATENT LITERATURE

PTL 1: International Publication No. 2012/035747

### NON-PATENT LITERATURE

NPL 1: Shiraishi et al. Clin. Cancer Res. 14, 1159-1166, 2008.

In order to effectively use hyperthermia, there is demand for various methods for improving the effects of hyperthermia.

It is an object of the present invention to improve the effects of hyperthermia on a cancer.

In order to achieve the object of the present invention, a pharmaceutical composition according to the present invention is characterized by comprising a chemokine and being for use in suppressing or preventing cancer metastasis, wherein the cancer is locally heated in the hyperthermia, and wherein the pharmaceutical composition is administered to a patient in combination with the hyperthermia.

The present invention relates to the embodiments as defined in the appended claims.

It is possible to improve the effects of hyperthermia on a cancer.

Other features and advantages of the present invention will be apparent from the following description taken in conjunction with the accompanying drawings. Note that the same reference numerals denote the same or like components throughout the accompanying drawings.

The accompanying drawings, which are incorporated in and constitute a part of the specification, illustrate embodiments of the invention and, together with the description, serve to explain principles of the invention.
FIG. 1 shows the amino acid sequence of a polypeptide eMIP according to an embodiment.
FIG. 2 shows changes in volume of mouse cancer cells over time in Example 1.
FIG. 3 shows a comparison between the volumes of mouse cancer cells in Example 1.
FIG. 4 shows changes in volume of mouse cancer cells over time in Example 2.
FIG. 5 shows a comparison between the volumes of mouse cancer cells in Example 2.
FIG. 6 shows a comparison between the numbers of metastatic colonies in mouse lungs in Example 3.
FIG. 7 shows metastatic colonies in left superior lobes of mouse lungs in Example 3.

Hereinafter, embodiments will be described in detail with reference to the attached drawings. Furthermore, the same reference numerals are given to the same or similar configurations, and redundant description thereof is omitted.

A pharmaceutical composition according to an embodiment described herein is a pharmaceutical composition for improving an effect of hyperthermia on a cancer, comprising a chemokine.

### Hyperthermia

Hyperthermia is a method for treating a cancer by heating a body or a portion thereof. In the hyperthermia, the whole body of a patient may be heated, or a cancer present in a portion of the body of a patient may be locally heated. For example, there is no particular limitation on a target site of the hyperthermia, and a portion that includes a solid cancer may be heated.

There is no particular limitation on the temperature at which the hyperthermia is performed, and the temperature may be 38°C or higher or 41°C or higher, for example. From the viewpoint that human cells including tumors are killed rapidly when the temperature thereof is higher than 42.5°C, the temperature may also be 42°C or higher, 42.5°C or higher, or 43°C or higher. Also, the temperature may be 100°C or lower, 60°C or lower, 50°C or lower, or 45°C or lower. There is also no particular limitation on the heating time. For example, the heating time may be 5 minutes or longer or 15 minutes or longer. Also, the heating time may be 3 hours or shorter or 1 hour or shorter.

The temperature at which the hyperthermia is performed means a temperature of a body or a portion thereof after heating. For example, when a portion of a body is locally heated, the temperature at which the hyperthermia is performed means a temperature of a treatment target site that has been raised through the hyperthermia. In this case, the treatment target site can be heated while the temperature of this site is being monitored. Moreover, when a whole body is heated, the temperature of the body after heating may be a temperature of a device used to heat the body, such as a temperature of a hot water bath.

There is no particular limitation on the heating method in the hyperthermia. For example, a cancer may be directly heated through heat conduction, or a cancer may be heated using electromagnetic waves or ultrasonic waves. When a cancer is heated through heat conduction, a high-temperature substance (e.g., a porous substance with high specific heat such as silica gel that has been heated) may be locally pressed against the cancer. In one embodiment, an affected site is irradiated with electromagnetic waves. There is no particular limitation on a specific method for using electromagnetic waves for heating. For example, an affected site is heated by applying electromagnetic waves between two electrodes. In this case, the affected site can be heated due to dielectric loss caused by delayed orientation polarization of molecules included in a dielectric substance in a region that includes the affected site. The electrodes may include an insulator layer in order to cause dielectric heating. Here, there is no particular limitation on the shapes of the electrodes. For example, the electrodes may have a plate-like shape, or may be probes. Heating can also be performed by arranging probe electrodes near an affected site (e.g., inserting probe electrodes into a body to an affected site) and allowing the electrodes to emit electromagnetic waves. There is no particular limitation on the type of electromagnetic wave, and microwaves, infrared rays, far infrared rays, or radio-frequency waves may be used.

Microwaves refer to electromagnetic waves that are defined as having a frequency of 300 MHz to 300 GHz. For example, microwaves having frequencies of 430 MHz, 915 MHz, and 2450 MHz are often used in a heating apparatus such as a microwave oven, but there is no particular limitation to such microwaves.

Radio-frequency waves are also called high-frequency waves, and refer to electromagnetic waves of 30 kHz to 300 MHz. There is no particular limitation on the range of the frequency to be used. For example, radio-frequency waves having a relatively low frequency of 1 MHz or less can be used, and radio-frequency waves having a relatively high frequency of 1 MHz or more can also be used. Heating through irradiation with radio-frequency waves can be performed by applying radio-frequency waves between two electrodes as described above. Compared with heating employing microwaves, heating employing radio-frequency waves is suitable for treatment of a relatively large tumor because a deeper position of a target can be heated due to the length of their wavelengths.

In one embodiment, an affected site is irradiated with ultrasonic waves. There is no particular limitation on a specific method for using ultrasonic waves for heating. There is no particular limitation on the type of ultrasonic wave to be used for heating, and high-intensity focused ultrasound (HIFU) can be used, for example. Using high-intensity focused ultrasound makes it possible to focus ultrasonic waves on one point and heat a local region. Heating employing high-intensity focused ultrasound may be performed by allowing a probe inserted into an affected site to emit high-intensity focused ultrasound, for example. For example, in the treatment of a prostate cancer, a probe that is inserted into the rectum through the anus may emit ultrasonic waves toward a region that includes the prostate cancer to heat this region.

### Chemokine

A chemokine is one type of cytokine that is a protein factor mediating intercellular interaction. Chemokines can act on leukocytes, lymphocytes, or the like. For example, chemokines can allow leukocytes, lymphocytes, or the like to migrate. Chemokines are classified into four types, namely CC, CXC, C, and CX3C, in accordance with a difference in a cysteine sequence included in chemokines. Also, chemokines can be classified into inflammatory chemokines and homeostatic chemokines. There is no particular limitation on the type of chemokine contained in the pharmaceutical composition. In one embodiment, a CC chemokine can be used, or an inflammatory chemokine can be used.

It should be noted that the chemokine may be a native chemokine derived from organisms such as humans, or a variant, fragment, or a derivative of the native chemokine. The variant as used herein refers to a protein having 90% of the amino acid sequence of the original protein with deletion, substitution, addition, and/or insertion of an amino acid. The fragment refers to a protein in which a continuous portion corresponding to 80% or less of the amino acid sequence of the original protein or a variant thereof is deleted and that has activity to improve the effects of the hyperthermia, similar to that of the original protein or a variant thereof. The fragment may also be a protein having an amino acid sequence that includes the amino acid sequence of an active site (a portion that includes an α-helix or β-sheet, for example, and binds to a receptor) and in which at least a portion of the amino acid sequence of a site other than the active site is deleted. Such a fragment can be found based on the results of three-dimensional structural analysis. The derivative refers to the original protein or a variant thereof, or a fragment thereof, that has been subjected to side-chain modification or modification such as PEGylation.

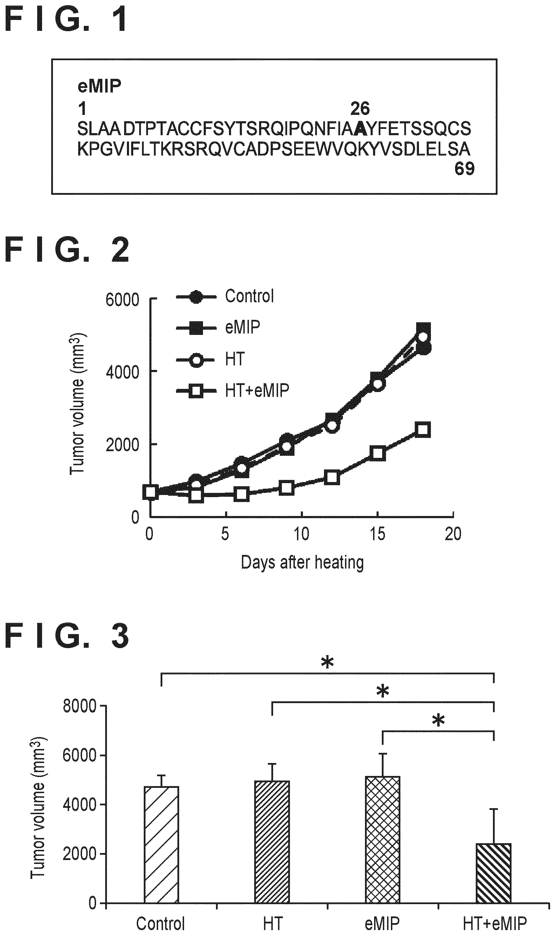

In one embodiment, CCL3 (SEQ ID No. 1), which is classified as a CC and inflammatory chemokine, can be used as the chemokine. Specifically, CCL, a CCL3 variant, a CCL3 fragment, or a CCL derivative can be used as the chemokine. The CCL3 variant may have an amino acid sequence consisting of the amino acid sequence of CCL in which aspartic acid at position 27 (from N-terminus) is substituted with alanine. The CCL3 variant may also have an amino acid sequence consisting of the amino acid sequence of CCL3 in which alanine at position 1 (from N-terminus) is deleted. The CCL3 derivative may include both of the two mutations above. In one embodiment, a CCL3 variant eMIP (SEQ ID No. 2) is used as the chemokine. The sequence of eMIP is also shown in FIG. 1. eMIP is also a variant of a macrophage inflammatory protein (MIP-1α), which is one of chemotactic proteins that are present in the human body and have immunostimulatory activity. eMIP has an amino acid sequence that includes 69 amino acids and consists of the amino acid sequence of MIP-1 α in which aspartic acid at position 26 is substituted with alanine.

A protein having a specific sequence, such as eMIP, can be produced and purified using a genetic engineering procedure that is well known to a person skilled in the art. For example, if DNA coding for a protein is introduced into a host cell, the protein can be produced using a biosynthesis system of the host cell. There is no particular limitation on the host cell used for this purpose, and well-known bacteria, yeasts, insect cells, animal cells, and plant cells may be used. Moreover, the method for introducing DNA into a host cell is adapted to the type of host cell. For example, such DNA is introduced into an animal cell using a phage or the like as a vector, or is introduced into a plant cell using an agrobacterium or the like as a vector or using a known particle gun method. These gene engineering procedures are known techniques, and thus specific descriptions thereof are omitted.

An expressed polypeptide can be obtained by performing a known protein purification method on the culture supernatant of the host cell obtained in the process above. For example, the protein can be produced through separation by precipitation and filtration. There is no particular limitation on the method of separation by precipitation and the filtration. For example, the separation by precipitation may be performed through salting out using ammonium sulfate, or by using an organic solvent such as ethanol, methanol, or acetone. The filtration may be performed through chromatography such as ion-exchange chromatography or isoelectric point chromatography, or microfiltration, ultrafiltration, or reverse osmosis filtration. Alternatively, two or more of these techniques may be used in combination. These protein purification methods are known techniques, and thus specific descriptions thereof are omitted.

A protein having a specific sequence, such as eMIP, can be produced using a chemical protein synthesis method that is well known to a person skilled in the art. For example, a protein can be synthesized using an Fmoc solid phase synthesis method. Moreover, a protein can be synthesized by synthesizing peptide portions included in the protein and linking the synthesized peptide portions together. The synthesized protein or peptide can be purified using the above-described methods. The Fmoc solid phase synthesis method is a known technique, and thus specific description thereof is omitted.

### Administration Method

As described above, the pharmaceutical composition according to this embodiment improves the effects of the hyperthermia on cancers. Accordingly, administration of the pharmaceutical composition according to this embodiment to a patient is performed in combination with the hyperthermia. There is no particular limitation on the method of using the pharmaceutical composition and the hyperthermia in combination. For example, the pharmaceutical composition may be administered after the hyperthermia. The hyperthermia may be repeated, and in this case, the pharmaceutical composition may be administered after each round of the hyperthermia. On the other hand, the pharmaceutical composition may be administered before the hyperthermia.

There is no particular limitation on the frequency and the number of times of administration of the pharmaceutical composition according to one embodiment, the dose thereof, and the like. For example, the pharmaceutical composition may be administered only once, or may be repeatedly administered. Moreover, the pharmaceutical composition may be administered once a day, twice or more a day, or once in every two or more days. The administration term of the pharmaceutical composition may be 3 days or more and 7 days or less, or 7 days or more. In one embodiment, the pharmaceutical composition is repeatedly administered after one round of the hyperthermia. The dose of the pharmaceutical composition may be 0.25 µg or more, 0.5 µg or more, or 1 µg or more. On the other hand, the dose of the pharmaceutical composition may be 100 mg or less or 10 mg or less. The number of times of administration of the pharmaceutical composition as described above may be adjusted as appropriate in accordance with the administration target, the size of a tumor, the treatment stage, the type of pharmaceutical composition, or the like. For example, when a patient is a human, the dose of the pharmaceutical composition may be 25 µg/kg or more, 50 µg/kg or more, or 82.5 µg/kg or more, and may be 200 µg/kg or less, 175 µg/kg or less, or 125 µg/kg or less.

The pharmaceutical composition can be administered through oral administration or parenteral administration. There is no particular limitation on the parenteral administration method, and examples thereof include intravenous administration, intra-arterial administration, local injection administration, intraperitoneal or intrathoracic administration, subcutaneous injection, intramuscular administration, sublingual administration, percutaneous absorption, and intrarectal administration. The dosage form of the pharmaceutical composition can be selected in accordance with the administration route, and an example thereof is an injection. The pharmaceutical composition may further include a substance having pharmaceutical activity other than the chemokine or a substance having no pharmaceutical activity. For example, the pharmaceutical composition can be produced using a commonly used excipient, filler, binder, wetting agent, disintegrator, surfactant, lubricant, dispersant, buffer, preservative, solubilizer, antiseptic, coloring agent, flavoring agent, stabilizer, or the like.

There is no particular limitation on the type of cancer from which a patient suffers and that is to be treated using the pharmaceutical composition. For example, the cancer may be a solid cancer. A solid cancer refers to a group of tumor cells that proliferate as a multicellular mass supported by a blood vessel. The solid cancer may be an epithelial cancer or a non-epithelial cancer. An epithelial cancer is a carcinoma generated on the skin or the epithelium such as a mucous membrane of a stomach or an intestine. Examples of the epithelial cancer include oral cancer, esophageal cancer, stomach cancer, colorectal cancer, prostate cancer, hepatic cancer, lung cancer, breast cancer, cervical cancer, and pancreatic cancer. The non-epithelial cancer is a sarcoma generated in bones throughout the body or soft tissues such as fat, muscles, and nerves. Examples of the non-epithelial cancer include retroperitoneal sarcoma, soft part sarcoma, uterine sarcoma, truncal sarcoma, and head and neck sarcoma. In one embodiment, the pharmaceutical composition is used to treat such a solid cancer, particularly prostate cancer, head and neck tumor, hepatic cancer, lung cancer, recurrent intramedullary cancer after a colorectal cancer operation, bone soft part tumor, cervical cancer or pancreatic cancer.

There is no particular limitation on a target patient of the treatment with the pharmaceutical composition. The patient may be a human suffering from a cancer as described above, and a mammal other than a human, such as a domestic animal (cow, horse, goat, sheep, and pig) and a pet (cat and dog).

With a treatment method according to one embodiment, a cancer of a patient can be treated by performing administration of the above-mentioned pharmaceutical composition containing a chemokine to a patient in combination with the hyperthermia. This pharmaceutical composition can improve the cancer treatment effects of the hyperthermia. Here, the treatment of a cancer encompasses delaying proliferation of a tumor and reducing the size of a tumor or the number of tumor cells. In one embodiment, a cancer found in a certain position in the body of a patient through an inspection (e.g., a primary cancer) can be treated using this treatment method. Moreover, in one embodiment, metastasis of a cancer in the body of a patient that has been subjected to the hyperthermia can be suppressed using this treatment method. For example, with one embodiment, micrometastasis of a tumor in the body of a patient can be killed by performing administration of the pharmaceutical composition in combination with the hyperthermia, thus making it possible to suppress or prevent the development of cancer metastasis.

Compared with the case where only the hyperthermia is performed, using the pharmaceutical composition according to one embodiment and the hyperthermia in combination makes it possible to improve the anti-tumor effect. For example, using the pharmaceutical composition and the hyperthermia in combination makes it possible to obtain synergistic anti-tumor activity. Accordingly, such a pharmaceutical composition can also be referred to as a "hyperthermia sensitizer".

### Examples

### Example 1

Six-week-old female BALB/c mice, eMIP (SEQ ID No. 2) serving as the pharmaceutical composition, and adenocarcinoma Colon 26 cells serving as cancer cells were prepared. First, 1×10⁵ Colon 26 cells were subcutaneously transplanted to the hind legs of each mouse. Then, when the diameter of the transplanted tumor was about 11 mm after 9 days, the mice were classified into four groups such that there was little difference in the tumor volume within each group.

The mice in one group were irradiated with radio-frequency waves using a hyperthermia apparatus (manufactured by Yamamoto Vinita Co., Ltd.), and were thus subjected to hyperthermic treatment such that the tumor site was heated only once at 42°C for 30 minutes. eMIP (2 µg/200 µl) was administered through the caudal vein once a day for 5 consecutive days from the next day. Then, a change in the tumor volume was measured after predetermined days elapsed since the hyperthermic treatment had been performed (HT + eMIP group).

In addition, as control experiments, a change in the tumor volume was measured regarding a mouse group that was subjected to only the transplantation of the cancer cells (Control group), a mouse group that was subjected to only hyperthermic treatment in the same manner as the HT + eMIP group after the transplantation of the cancer cells (HT group), and a mouse group that was subjected to only the administration of eMIP in the same manner as the HT + eMIP group after the transplantation of the cancer cells (eMIP group).

FIG. 2 shows the measurement results. As shown in FIG. 2, in the HT group, the tumor volume did not change when only the hyperthermic treatment was performed, and the subsequent increase in the tumor volume was the same as that in the Control group. Also, in the eMIP group, the tumor volume did not change when only the administration of eMIP was performed, and the subsequent increase in the tumor volume was the same as that in the Control group. However, in the HT + eMIP group, the increase in the tumor volume was reduced compared with the HT group, the eMIP group, and the Control group.

FIG. 3 shows the tumor volumes in the groups 18 days after the hyperthermic treatment. As shown in FIG. 3, the tumor volume in the HT + eMIP group was obviously and statistically significantly smaller than those in the Control group, the HT group, and the eMIP group (P<0.05 (ANOVA)). It was shown from these results that the anti-tumor activity was synergistically improved by using the hyperthermia and the administration of a chemokine in combination.

### Example 2

Seven-week-old male BALB/c mice that were purchased when six weeks old and then were kept and acclimated, eMIP (SEQ ID No. 2) serving as the pharmaceutical composition, and adenocarcinoma Colon 26 cells serving as cancer cells were prepared. First, 2×10⁵ Colon 26 cells were subcutaneously transplanted to the right abdomen of each mouse. Then, when the volume of the transplanted tumor was about 250 mm³ after 16 days, the mice were classified into three groups such that there was little difference in the tumor volume within each group.

The mice in one group were subjected to hyperthermic treatment using a hyperthermia apparatus such that the tumor site was heated only once at 42°C for 30 minutes while the local temperature of the tumor site in the right abdomen was monitored using a disposable skin surface temperature probe (manufactured by Philips). eMIP (2.5 µg/200 µl) was administered through the caudal vein once a day for 5 consecutive days from the next day. Then, a change in the tumor volume was measured after predetermined days elapsed since the hyperthermic treatment had been performed (HT + eMIP group). In addition, as control experiments, a change in the tumor volume was measured regarding a mouse group that was subjected to only the transplantation of the cancer cells (Control group), and a mouse group that was subjected to only hyperthermic treatment in the same manner as the HT + eMIP group after the transplantation of the cancer cells (HT group). It should be noted that the tumor volume was calculated using the equation below. Tumor volume = long diameter of tumor × (short diameter of tumor)2 × 0.5236

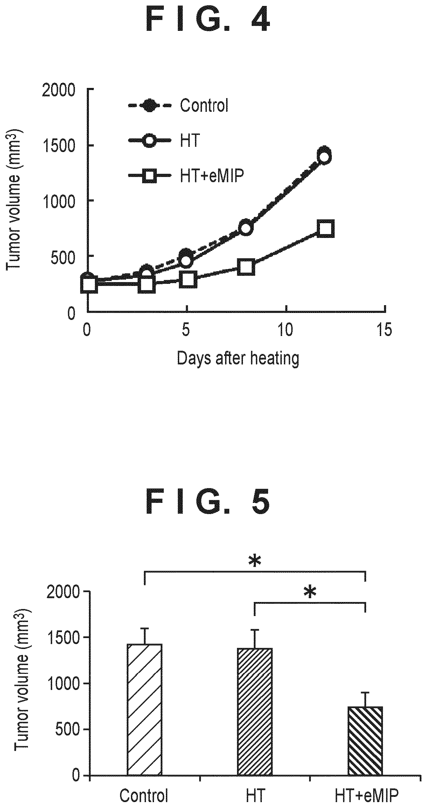

FIG. 4 shows the measurement results. As shown in FIG. 4, in the HT group, the tumor volume did not change when only the hyperthermic treatment was performed, and the subsequent increase in the tumor volume was the same as that in the Control group. However, in the HT + eMIP group, the increase in the tumor volume was reduced compared with the HT group and the Control group.

FIG. 5 shows the tumor volumes in the groups 12 days after the hyperthermic treatment. As shown in FIG. 5, the tumor volume in the HT + eMIP group was obviously and statistically significantly smaller than those in the Control group and the HT group (P<0.05 (ANOVA)). It was shown from these results that the anti-tumor activity was synergistically improved by performing the hyperthermia and the administration of a chemokine in combination on tumors in various sites of a body.

### Example 3

Six-week-old female BALB/c mice, eMIP (SEQ ID No. 2) serving as the pharmaceutical composition, and adenocarcinoma Colon 26 cells serving as cancer cells were prepared. First, 1×10⁵ Colon 26 cells were subcutaneously transplanted to the hind legs of each mouse. Then, when the diameter of the transplanted tumor was about 11 mm after 9 days, the mice were classified into four groups such that there was little difference in the tumor volume within each group.

The mice in one group were irradiated with radio-frequency waves using a hyperthermia apparatus (manufactured by Yamamoto Vinita Co., Ltd.), and were thus subjected to hyperthermic treatment such that the tumor site was heated only once at 42°C for 30 minutes. eMIP (2 µg/200 µl) was administered through the caudal vein once a day for 5 consecutive days from the next day. Then, the lung of each mouse was extracted 18 days after the hyperthermic treatment, and the number of metastatic colonies of the cancer cells in the lung was counted.

In addition, as control experiments, the number of metastatic colonies of the cancer cells in the lung was counted in the same manner regarding a mouse group that was subjected to only the transplantation of the cancer cells (Control group), a mouse group that was subjected to only hyperthermic treatment in the same manner as the HT + eMIP group after the transplantation of the cancer cells (HT group), and a mouse group that was subjected to only the administration of eMIP in the same manner as the HT + eMIP group after the transplantation of the cancer cells (eMIP group).

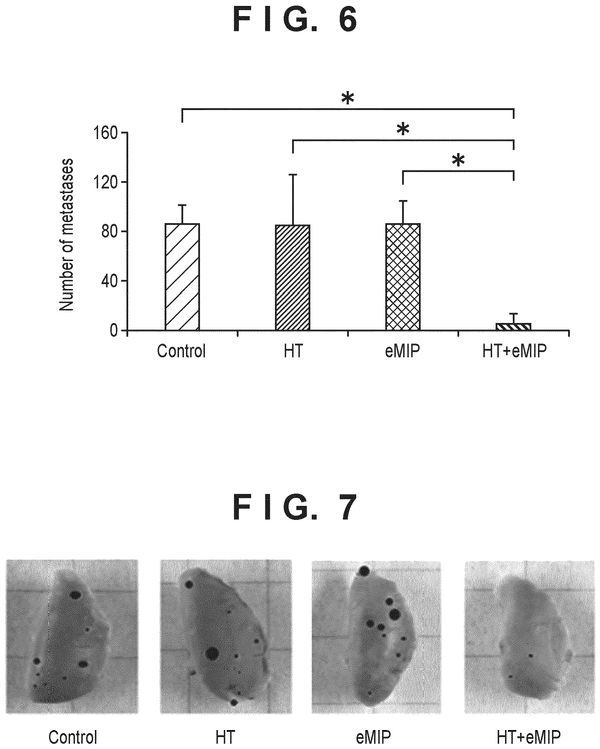

FIG. 6 shows the measurement results. As shown in FIG. 6, the number of metastatic colonies of the cancer cells in the lung in the HT + eMIP group was statistically significantly reduced compared with the Control group, the HT group, and the eMIP group (P<0.05 (ANOVA)). In addition, in the HT + eMIP group, metastatic colonies were not observed in half of the mice. FIG. 7 shows the left superior lobes of the lungs extracted from the mice of the respective groups. In FIG. 7, the outlines of the metastatic colonies were black in color. As is clear from FIG. 7, the number of metastatic colonies in the lung in the HT + eMIP group was smaller than those in the Control group, the HT group, and the eMIP group. It was shown from these results that significant effects to suppress metastasis of a cancer were obtained by using the hyperthermia and the administration of a chemokine in combination.

## Claims

1. A pharmaceutical composition comprising a chemokine for use in suppressing or preventing cancer metastasis
wherein the pharmaceutical composition is administered to a patient in combination with hyperthermia, wherein the cancer is locally heated in the hyperthermia.

2. The pharmaceutical composition for use according to claim 1, wherein the chemokine is CCL3, a CCL3 variant, a CCL3 fragment, or a CCL3 derivative.

3. The pharmaceutical composition for use according to claim 1 or 2, wherein the chemokine has an amino acid sequence consisting of an amino acid sequence of CCL3 in which aspartic acid at position 27 is substituted with alanine.

4. The pharmaceutical composition for use according to any one of claims 1 to 3, wherein the chemokine has an amino acid sequence consisting of an amino acid sequence of CCL3 in which alanine at position 1 is deleted.

5. The pharmaceutical composition for use according to any one of claims 1 to 4, wherein the chemokine is eMIP.

6. The pharmaceutical composition for use according to any one of claims 1 to 5, wherein the cancer is heated to a temperature of 41°C or higher in the hyperthermia.

7. The pharmaceutical composition for use according to any one of claims 1 to 6, wherein the cancer is heated using electromagnetic waves or ultrasonic waves in the hyperthermia.

8. The pharmaceutical composition for use according to claim 7, wherein the cancer is irradiated with microwaves, infrared rays, far infrared rays, or radio-frequency waves in the hyperthermia.

9. The pharmaceutical composition for use according to claim 7, wherein the cancer is irradiated with high-intensity focused ultrasound (HIFU) in the hyperthermia.

10. The pharmaceutical composition for use according to any one of claims 1 to 9, wherein the pharmaceutical composition is administered after the hyperthermia.

11. The pharmaceutical composition for use according to any one of claims 1 to 10, wherein the pharmaceutical composition is repeatedly administered.

12. The pharmaceutical composition for use according to any one of claims 1 to 11, wherein the cancer is prostate cancer, head and neck tumor, hepatic cancer, lung cancer, recurrent intramedullary cancer after a colorectal cancer operation, bone soft part tumor, cervical cancer or pancreatic cancer.

13. The pharmaceutical composition for use according to any one of claims 1 to 12, wherein, in the hyperthermia in which the cancer is locally heated, the cancer is locally heated to a temperature of 45°C or lower.

14. The pharmaceutical composition for use according to any one of claims 1 to 13, wherein, in the hyperthermia in which the cancer is locally heated, heating employing heat conduction is performed.

15. The pharmaceutical composition for use according to claim 14, wherein, in the hyperthermia in which the cancer is locally heated, the cancer is heated through heat conduction from a high temperature substance.

16. The pharmaceutical composition for use according to claim 14 or 15, wherein the heating employing heat conduction is performed by pressing a porous substance with high specific heat that has been heated against an affected site.

17. The pharmaceutical composition for use according to any one of claims 1 to 16, wherein, in the hyperthermia in which the cancer is locally heated, heating is performed for 5 minutes or longer.

18. The pharmaceutical composition for use according to any one of claims 1 to 17, wherein, in the hyperthermia in which the cancer is locally heated, heating is performed for 1 hour or shorter.

19. The pharmaceutical composition for use according to any one of claims 1 to 18, wherein, in the hyperthermia in which the cancer is locally heated, heating is performed while a temperature of a heated site is being monitored.

20. The pharmaceutical composition for use according to any one of claims 1 to 14, wherein, in the hyperthermia in which the cancer is locally heated, heating is performed using a probe inserted to a position near an affected site, wherein the probe emits electromagnetic waves or ultrasonic waves.

21. The pharmaceutical composition for use according to any one of claims 1 to 20, wherein, in the hyperthermia in which the cancer is locally heated, a region that includes an affected site is heated.

22. The pharmaceutical composition for use according to any one of claims 1 to 14, wherein, in the hyperthermia in which the cancer is locally heated, a region that includes an affected site is heated using a probe inserted to a position near the affected site, wherein the probe emits electromagnetic waves or ultrasonic waves.

## Patentansprüche

1. Pharmazeutische Zusammensetzung, umfassend ein Chemokin, zur Verwendung bei der Unterdrückung oder Verhinderung der Metastasierung von Krebs,
wobei die pharmazeutische Zusammensetzung einem Patienten in Kombination mit Hyperthermie verabreicht wird, wobei der Krebs in der Hyperthermie lokal erhitzt wird.

2. Pharmazeutische Zusammensetzung zur Verwendung nach Anspruch 1, wobei das Chemokin CCL3, eine CCL3-Variante, ein CCL3-Fragment oder ein CCL3-Derivat ist.

3. Pharmazeutische Zusammensetzung zur Verwendung nach Anspruch 1 oder 2, wobei das Chemokin eine Aminosäuresequenz aufweist, bestehend aus einer Aminosäuresequenz von CCL3, in der Asparaginsäure an Position 27 durch Alanin ersetzt ist.

4. Pharmazeutische Zusammensetzung zur Verwendung nach einem der Ansprüche 1 bis 3, wobei das Chemokin eine Aminosäuresequenz aufweist, bestehend aus einer Aminosäuresequenz von CCL3, bei der Alanin an Position 1 deletiert ist.

5. Pharmazeutische Zusammensetzung zur Verwendung nach einem der Ansprüche 1 bis 4, wobei das Chemokin eMIP ist.

6. Pharmazeutische Zusammensetzung zur Verwendung nach einem der Ansprüche 1 bis 5, wobei der Krebs in der Hyperthermie auf eine Temperatur von 41°C oder höher erhitzt wird.

7. Pharmazeutische Zusammensetzung zur Verwendung nach einem der Ansprüche 1 bis 6, wobei der Krebs in der Hyperthermie unter Verwendung von elektromagnetischen Wellen oder Ultraschallwellen erhitzt wird.

8. Pharmazeutische Zusammensetzung zur Verwendung nach Anspruch 7, wobei der Krebs in der Hyperthermie mit Mikrowellen, Infrarotstrahlen, Ferninfrarotstrahlen oder Radiofrequenzwellen bestrahlt wird.

9. Pharmazeutische Zusammensetzung zur Verwendung nach Anspruch 7, wobei der Krebs in der Hyperthermie mit hochintensivem fokussiertem Ultraschall *(high-intensity focused ultrasound;* HIFU) bestrahlt wird.

10. Pharmazeutische Zusammensetzung zur Verwendung nach einem der Ansprüche 1 bis 9, wobei die pharmazeutische Zusammensetzung nach der Hyperthermie verabreicht wird.

11. Pharmazeutische Zusammensetzung zur Verwendung nach einem der Ansprüche 1 bis 10, wobei die pharmazeutische Zusammensetzung wiederholt verabreicht wird.

12. Pharmazeutische Zusammensetzung zur Verwendung nach einem der Ansprüche 1 bis 11, wobei der Krebs Prostatakrebs, Kopf- und Halstumor, Leberkrebs, Lungenkrebs, rezidivierender intramedullärer Krebs nach einer Darmkrebsoperation, Knochenweichteiltumor, Gebärmutterhalskrebs oder Pankreaskrebs ist.

13. Pharmazeutische Zusammensetzung zur Verwendung nach einem der Ansprüche 1 bis 12, wobei in der Hyperthermie, bei welcher der Krebs lokal erhitzt wird, der Krebs lokal auf eine Temperatur von 45°C oder weniger erhitzt wird.

14. Pharmazeutische Zusammensetzung zur Verwendung nach einem der Ansprüche 1 bis 13, wobei in der Hyperthermie, bei welcher der Krebs lokal erhitzt wird, eine Erhitzung unter Verwendung von Wärmeleitung durchgeführt wird.

15. Pharmazeutische Zusammensetzung zur Verwendung nach Anspruch 14, wobei in der Hyperthermie, bei welcher der Krebs lokal erhitzt wird, der Krebs durch Wärmeleitung von einer Hochtemperatursubstanz erhitzt wird.

16. Pharmazeutische Zusammensetzung zur Verwendung nach Anspruch 14 oder 15, wobei die Erhitzung unter Verwendung von Wärmeleitung durch Pressen einer porösen Substanz mit hoher spezifischer Wärme, die erhitzt wurde, gegen eine betroffene Stelle erfolgt.

17. Pharmazeutische Zusammensetzung zur Verwendung nach einem der Ansprüche 1 bis 16, wobei in der Hyperthermie, bei welcher der Krebs lokal erhitzt wird, die Erhitzung für 5 Minuten oder länger durchgeführt wird.

18. Pharmazeutische Zusammensetzung zur Verwendung nach einem der Ansprüche 1 bis 17, wobei in der Hyperthermie, bei welcher der Krebs lokal erhitzt wird, die Erhitzung für 1 Stunde oder kürzer durchgeführt wird.

19. Pharmazeutische Zusammensetzung zur Verwendung nach einem der Ansprüche 1 bis 18, wobei in der Hyperthermie, bei welcher der Krebs lokal erhitzt wird, die Erhitzung durchgeführt wird, während eine Temperatur einer erhitzten Stelle überwacht wird.

20. Pharmazeutische Zusammensetzung zur Verwendung nach einem der Ansprüche 1 bis 14, wobei in der Hyperthermie, bei welcher der Krebs lokal erhitzt wird, die Erhitzung unter Verwendung einer Sonde durchgeführt wird, die in eine Position nahe einer betroffenen Stelle eingeführt wird, wobei die Sonde elektromagnetische Wellen oder Ultraschallwellen aussendet.

21. Pharmazeutische Zusammensetzung zur Verwendung nach einem der Ansprüche 1 bis 20, wobei in der Hyperthermie, bei welcher der Krebs lokal erhitzt wird, ein Bereich, der eine betroffene Stelle enthält, erhitzt wird.

22. Pharmazeutische Zusammensetzung zur Verwendung nach einem der Ansprüche 1 bis 14, wobei in der Hyperthermie, bei welcher der Krebs lokal erhitzt wird, ein Bereich, der eine betroffene Stelle enthält, unter Verwendung einer Sonde erhitzt wird, die in eine Position nahe der betroffenen Stelle eingeführt wird, wobei die Sonde elektromagnetische Wellen oder Ultraschallwellen aussendet.

## Revendications

1. Composition pharmaceutique comprenant une chimiokine destinée à être utilisée dans la suppression ou prévention de métastase cancéreuse,
dans laquelle la composition pharmaceutique est administrée à un patient en combinaison avec de l'hyperthermie, dans laquelle le cancer est chauffé localement dans l'hyperthermie.

2. Composition pharmaceutique destinée à être utilisée selon la revendication 1, dans laquelle la chimiokine est CCL3, un variant de CCL3, un fragment de CCL3 ou un dérivé de CCL3.

3. Composition pharmaceutique destinée à être utilisée selon la revendication 1 ou 2, dans laquelle la chimiokine a une séquence d'acides aminés constituée d'une séquence d'acides aminés de CCL3 dans laquelle l'acide aspartique à la position 27 est substitué par de l'alanine.

4. Composition pharmaceutique destinée à être utilisée selon l'une quelconque des revendications 1 à 3, dans laquelle la chimiokine a une séquence d'acides aminés constituée d'une séquence d'acides aminés de CCL3 dans laquelle l'alanine à la position 1 est délétée.

5. Composition pharmaceutique destinée à être utilisée selon l'une quelconque des revendications 1 à 4, dans laquelle la chimiokine est eMIP.

6. Composition pharmaceutique destinée à être utilisée selon l'une quelconque des revendications 1 à 5, dans laquelle le cancer est chauffé à une température de 41 °C ou supérieure dans l'hyperthermie.

7. Composition pharmaceutique destinée à être utilisée selon l'une quelconque des revendications 1 à 6, dans laquelle le cancer est chauffé en utilisant des ondes électromagnétiques ou ondes ultrasonores dans l'hyperthermie.

8. Composition pharmaceutique destinée à être utilisée selon la revendication 7, dans laquelle le cancer est irradié avec des micro-ondes, rayons infrarouge, rayons infrarouge lointain ou ondes de radiofréquence dans l'hyperthermie.

9. Composition pharmaceutique destinée à être utilisée selon la revendication 7, dans laquelle le cancer est irradié avec des ultrasons focalisés de haute intensité (HIFU) dans l'hyperthermie.

10. Composition pharmaceutique destinée à être utilisée selon l'une quelconque des revendications 1 à 9, dans laquelle la composition pharmaceutique est administrée après l'hyperthermie.

11. Composition pharmaceutique destinée à être utilisée selon l'une quelconque des revendications 1 à 10, dans laquelle la composition pharmaceutique est administrée de manière répétée.

12. Composition pharmaceutique destinée à être utilisée selon l'une quelconque des revendications 1 à 11, dans laquelle le cancer est un cancer de la prostate, une tumeur de la tête et du cou, un cancer hépatique, un cancer du poumon, un cancer intramédullaire récurrent après une opération de cancer colorectal, une tumeur des parties molles osseuses, un cancer du col de l'utérus ou un cancer pancréatique.

13. Composition pharmaceutique destinée à être utilisée selon l'une quelconque des revendications 1 à 12, dans laquelle le cancer est chauffé localement à une température de 45 °C ou inférieure dans l'hyperthermie dans laquelle le cancer est chauffé localement.

14. Composition pharmaceutique destinée à être utilisée selon l'une quelconque des revendications 1 à 13, dans laquelle un chauffage employant de la conduction thermique est réalisé dans l'hyperthermie dans laquelle le cancer est chauffé localement.

15. Composition pharmaceutique destinée à être utilisée selon la revendication 14, dans laquelle le cancer est chauffé par conduction thermique à partir d'une substance à haute température dans l'hyperthermie dans laquelle le cancer est chauffé localement.

16. Composition pharmaceutique destinée à être utilisée selon la revendication 14 ou 15, dans laquelle le chauffage employant de la conduction thermique est réalisé en pressant une substance poreuse avec une chaleur spécifique élevée qui a été chauffée contre un site affecté.

17. Composition pharmaceutique destinée à être utilisée selon l'une quelconque des revendications 1 à 16, dans laquelle le chauffage est réalisé pendant 5 minutes ou plus longtemps dans l'hyperthermie dans laquelle le cancer est chauffé localement.

18. Composition pharmaceutique destinée à être utilisée selon l'une quelconque des revendications 1 à 17, dans laquelle le chauffage est réalisé pendant 1 heure ou une durée plus courte dans l'hyperthermie dans laquelle le cancer est chauffé localement.

19. Composition pharmaceutique destinée à être utilisée selon l'une quelconque des revendications 1 à 18, dans laquelle le chauffage est réalisé tandis qu'une température d'un site chauffé est surveillée dans l'hyperthermie dans laquelle le cancer est chauffé localement.

20. Composition pharmaceutique destinée à être utilisée selon l'une quelconque des revendications 1 à 14, dans laquelle le chauffage est réalisé en utilisant une sonde insérée à une position près d'un site affecté dans l'hyperthermie dans laquelle le cancer est chauffé localement, dans laquelle la sonde émet des ondes électromagnétiques ou ondes ultrasonores.

21. Composition pharmaceutique destinée à être utilisée selon l'une quelconque des revendications 1 à 20, dans laquelle une région qui inclut un site affecté est chauffée dans l'hyperthermie dans laquelle le cancer est chauffé localement.

22. Composition pharmaceutique destinée à être utilisée selon l'une quelconque des revendications 1 à 14, dans laquelle une région qui inclut un site affecté est chauffée en utilisant une sonde insérée à une position près du site affecté dans l'hyperthermie dans laquelle le cancer est chauffé localement, dans laquelle la sonde émet des ondes électromagnétiques ou ondes ultrasonores.
